# EUROPEAN PATENT SPECIFICATION

(11) **EP 1 309 349 B1**
(45) Date of publication and mention of the grant of the patent: **09.11.2005**
(21) Application number: 01956518.3
(22) Date of filing: 09.07.2001
(51) Int. Cl.: A61K 45/06, A61P 31/14, A61P 31/18

(54) **PHARMACEUTICAL COMPOSITION AND METHOD FOR THE TREATMENT OF RETROVIRAL INFECTIONS**
PHARMAZEUTISCHE ZUSAMMENSETZUNG UND VERFAHREN ZUR BEHANDLUNG VON RETROVIRALEN INFEKTIONEN
COMPOSITION PHARMACEUTIQUE ET TECHNIQUE DE TRAITEMENT DES INFECTIONS RETROVIRALES

(30) Priority: 07.07.2000 GB 0016845; 21.09.2000 US 234416 P; 18.01.2001 US 262612 P
(43) Date of publication of application: 14.05.2003
(73) Proprietor: Stichting REGA VZW, 3000 Leuven (BE)
(72) Inventor: DE CLERCQ, Erik, B-3360 Lovenjoel (BE); DEBYSER, Zeger, B-3001 Heverlee (BE); PLYMERS, Willem, B-3001 Heverlee (BE); PANNECOUQUE, Christophe, B-3001 Heverlee (BE); WITVROUW, Myriam, B-3201 Langdorp (BE)
(74) Representative: Bird, Ariane
(86) International application number: PCT/EP2001/007882
(87) International publication number: WO 2002/003971

(56) References cited:
- WO-A-99/25718

## Description

### FIELD OF THE INVENTION

The present invention relates pharmaceutical compositions and their use for the treatment of retroviral infections in mammals. More specifically, it relates to the use of certain pyrano[2,3-d:6,5-d']dipyrimidine derivatives, for manufacturing a medicine for treating retroviral infections in mammals. In another embodiment, it relates to the use of these derivatives in combination with one or more drugs targeting one or more cellular or retroviral proteins, for instance retroviral enzyme inhibitors.

### BACKGROUND OF THE INVENTION

Replication of the human immunodeficiency virus type 1 (hereinafter referred as HIV-1) can be drastically reduced in infected patients by combining potent antiviral drugs targeted at multiple viral targets, as reviewed by Vandamme et al. in *Antiviral Chem. Chemother.* (1998) 9:187-203. Drugs that have been formally approved for the treatment of HIV-infected patients belong to the class of reverse transcriptase (RT) inhibitors (nucleosides and non-nucleosides) or protease inhibitors. Multiple-drug combination regimens can reduce viral load below the detection limit of the most sensitive tests. Nevertheless low level ongoing replication has been shown to occur, possibly in sanctuary sites, leading to the emergence of drug-resistant strains, according to Perelson et al. in *Nature* (1997) 387:123-124. Moreover, HIV can develop resistance to most, if not all, currently approved antiviral drugs, according to Schmit et al. in J. *Infect. Dis.* (1996) 174:962-968.

The only enzyme required for HIV-1 integration is integrase (hereinafter referred as IN), a protein of 32 kDa encoded at the 3'-end of the *pol* gene, as reviewed by Brown in *Retroviruses* (1997), Coffin, Hughes and Varmus Eds. (Cold Spring Harbor Laboratory Press USA) pp. 161-203. The enzyme is produced by protease-mediated cleavage of the gag-pol precursor during virion maturation. Integrase recognizes specific sequences in the long terminal repeat (hereinafter referred as LTR) elements of the viral cDNA. The terminal 15 bp of the LTR are necessary and sufficient for site-specific cleavage and integration. The highly conserved dinucleotide CA immediately upstream of the cleavage site, is critical for enzymatic activity. In the first step of the integration reaction, termed 3'-end processing, two nucleotides are removed from each 3'-end to produce new 3'-hydroxyl ends (CA-3'-OH). This reaction occurs in the cytoplasm within a large viral nucleoprotein complex called the pre-integration complex (hereinafter referred as PIC), according to Miller et al. in *J. Virol*. (1997) 71:5382-5390. After entering the nucleus, the processed viral double-stranded DNA is joined to host target DNA. The joining reaction includes a coupled 5 bp staggered cleavage of the target host DNA and the ligation of processed CA-3'-OH viral DNA ends to the 5'-O-phosphate ends of the target DNA. Repair of the remaining gaps, although not understood at this time, is probably accomplished by host-cell DNA repair enzymes although retroviral enzymes were assumed to be implicated as well according to Chow et al. in *Science* (1992) 255:723-726.

Based on partial proteolysis experiments, HIV-IN can be divided into three functional domains. The N-terminal domain (comprising residues 1 to 50) is characterized by a HHCC "zinc finger"-like sequence (i.e. contains 2 histidines and 2 cysteines that bind Zn²⁺) but its exact function remains unknown. The central core domain (comprising residues 50 to 212) is characterized by three amino acid residues (Asp64, Asp116 and Glu152) that are highly conserved in the integrase superfamily and polynucleotide transferases and that represent the catalytic domain for both 3'-processing and DNA strand-transfer activities. This central core domain alone can carry out an apparent reversal of the DNA strand-transfer reaction *in vitro*, the so-called disintegration reaction. However, both the amino- and the carboxy-terminal domains are necessary for catalysis of 3'-processing and strand-transfer. A single amino acid substitution (F185K) within the catalytic core domain generates a soluble protein that has enabled the core domain to be crystallized and the three-dimensional (hereinafter referred as 3D) structure to be resolved, as reviewed by Dyda et al. in *Science* (1994) 266:1981-1986. The C-terminal domain (comprising residues 212 to 288) is involved in non-specific DNA-binding. Nuclear magnetic resonance (hereinafter referred as NMR) structures of a truncated C-terminal domain have been determined. Complementation studies with IN proteins mutated in the different domains suggest that the active form of IN is an oligomer, although the exact stoeichiometry remains unknown.

Establishment of high-throughput microtiter plate assays and the elucidation of the 3D structure of the catalytic domain of HIV-1 IN helped the development of IN inhibitors by screening chemical libraries or by structure-based design. Although evaluation of IN inhibitors in rather cumbersome PIC assays has been discussed (e.g. by Farnet et al. in *Proc. Natl. Acad. Sci USA* (1996) 93:9742-9747), IN inhibition is typically assessed in oligonucleotide-based assays using LTR mimics to evaluate both processing and joining reactions *in vitro* such as disclosed namely by Sherman et al. in *Proc. Natl. Acad. Sci. USA* (1990) 87:5119-5123 and by Bushman et al. in *Proc. Natl. Acad. Sci. USA* (1991) 88 :1339-1343. Different classes of HIV-1 IN inhibitors have been reported, as reviewed by Y. Pommier et al. in *Antiviral Research* (2000) 47:139-148, and include (i) mono-, di-, iso- and longer oligonucleotides, and analogues thereof, (ii) hydroxylated aromatic compounds, (iii) DNA-interacting agents (ligands) and (iv) polypeptides and antibodies. However, most of these compounds did not exhibit antiviral activity in cell culture. For most of the IN inhibitors that did show antiviral activity in cell culture, it was not unambiguously demonstrated that the integration step was targeted. The only authentic IN selective inhibitors reported so far are diketo acid derivatives, according to Hazuda et al. in *Science* (2000) 287:646-650.

Nucleotides such as 3',5-diazido-2',3'-dideoxyuridine 5'-monophosphate (referred as 5-N₃-AZUMP) interfere with the enzymatic activity of IN likely through an interaction with the nucleotide binding site, more precisely through binding to the α-4 helix of the HIV-1 core domain. Structure-activity studies of various nucleotide analogues have been reported. Although the concentration required for inhibition was high (IC₅₀ = 150 µM), it could not be excluded that IN inhibition may contribute to the antiviral effect of AZT, since concentrations up to 1 mM 5-N₃-AZUMP can accumulate in cells according to Furman et al. in *Proc. Natl. Acad. Sci. USA* (1986) 83:8333-7. Dinucleotides were also reported as potent inhibitors of HIV-1 IN, even in the absence of 5'-O-phosphorylation, yet none showed antiviral activity as reported by Mazumder et al. in *Mol. Pharmacol*. (1997) 51:567-75.

Oligonucleotides that form four-stranded guanosine quartet structures are potent inhibitors of HIV replication in cell culture (according to Ojwang et al. in *Antimicrob. Agents Chemother.* (1995) 39:2426-2435) but, although G-quartets inhibit HIV IN activity in the nanomolar range, it has been clearly shown (by Cherepanov et al. in *Mol. Pharmacol*. (1997) 52:771-780) by selecting and sequencing drug-resistant strains that their antiviral activity in cell culture is due to the inhibition of viral entry rather than HIV IN activity.

Many polyhydroxylated aromatic compounds (including flavones, tyrphostins, lignans, anthraquinones and biscatechols, all characterized by two vicinal hydroxyl groups on an aromatic ring) have been reported as inhibitors of HIV-1 IN *in vitro.* Three possible mechanisms of action were proposed (see Pommier et al. cited *supra).* Catechols may interfere with the coordination of metal ions required for the phosphoryl transfer reactions in the active site. Oxidation of catechols *in vivo* could yield reactive quinone species or the hydroxyl groups may function as hydrogen bond donors for interaction with the enzyme. Hydroxylated aromatics that do not contain ortho hydroxyl groups, such as curcumin, coumermycin and bis-coumarins, but inhibit IN activity have been reported as well. Using molecular modelling, pharmacophores have been identified among the known hydroxylated aromatic inhibitors that may lead to discovery of novel lead compounds, according to Neamati et al. in *Mol. Pharmacol.* (1997) 52:1041-1055. However, antiviral potency of catechol-type inhibitors in cell culture is impaired by inherent cellular toxicity.

Dicaffeoylquinic acid and L-chicoric acid (L-CA) derivatives were recently reported by Robinson et al. in *Proc. Natl. Acad. Sci. USA* (1996) 93:6326-6331 to inhibit HIV-1 IN *in vitro* and HIV-1 replication in cell culture. An HIV-1 strain resistant to L-chicoric acid was selected and a mutation was mapped to the IN gene (G140S). We have recently selected HIV-1 strains that are resistant to L-CA and contain mutations in *gp120* instead of the *IN* gene. Moreover, recombinant IN carrying the G140S mutation was inhibited to the same extent by L-CA as the wild type enzyme, according to Pluymers et al. (2000).

Diketo acid derivatives inhibit HIV-1 replication at micromolar concentrations through a specific inhibition of the DNA strand-transfer step, as reported by Hazuda et al. (cited *supra).* Resistant virus strains selected in the presence of these compounds, carried mutations in the IN gene. When introduced in IN, these mutations conferred partial resistance to the drugs.

Many DNA binding agents were also found to inhibit HIV-1 IN, probably due to a non-specific interaction with the DNA binding domain of the enzyme. DNA intercalators and DNA groove binders such as netropsin belong to this category. DNA ligands forming triple helices in specific regions of the LTR, e.g. through linkage to an intercalating oxazolopyridocarbazole can act as U3-specific or U5-specific LTR binding inhibitors of HIV-1 IN. A 30-mer peptide corresponding to amino-acids 147 to 175 of HIV-1 IN was also shown to inhibit IN activity at milfimolar concentrations. Intracellular expression of single-chain variable fragments against IN domains inhibit early stages of the viral replication cycle. Polypeptides such as members of the family of ribosome inactivating proteins (RIP's) were described as potent inhibitors of HIV-1 IN, however it was not demonstrated that interference with IN is the key mechanism for their antiviral activity.

International patent publication WO 99/25718 discloses a class of derivatives of pyrano[2,3-d:6,5-d'] dipyrimidine which are said to be useful in pharmacology, veterinary and cosmetology, in particular having antimicrobial, antiviral (relative to Herpes simplex virus), antichlamydial and immune stimulating (as inducers of interferon) activities and to be useful for treating opportunistic diseases in patients suffering from immunodeficiency.

As a summary, there is still a stringent need in the art for potent inhibitors selectively targeting retroviral RT and/or IN and being also antiviral. Therefore a goal of the present invention is to satisfy this urgent need by identifying efficient and non-harmful pharmaceutically active ingredients and combination of ingredients for the treatment of retroviral infections, in particular lentiviral infections, and more particularly HIV infections, in mammals and in humans.

### SUMMARY OF THE INVENTION

In an effort to improve therapy of retroviral infections, in particular lentiviral infections, and more particularly HIV infections, in mammals and in humans, to reduce viral replication even further and to cope with retrovirus strains that are resistant to multiple drugs, it was desirable to initiate a search for effective inhibitors of the third viral enzyme, the integrase (HIV-IN). This enzyme is responsible for inserting the viral cDNA into the host cell chromosome, an essential step for the replication of the virus. Since no human counterpart of the enzyme is yet known, there is considerable interest in developing effective and preferably selective inhibitors of the HIV integration process.

The present invention is based on the unexpected discovery of the inhibition of retroviral, in particular HIV-1, integrase activity by certain derivatives of pyrano[2,3-d:6,5-d'] dipyrimidine, the said compounds also showing protection against replication of HIV in cell culture and also being able to show a synergistic effect against a retroviral infection (in particular a lentiviral infection) in mammalian and human cell culture when used in combination with one or more anti-retroviral drugs, e.g. drugs effective against one or more retroviral or cellular proteins involved in the entry and/or replication of a retrovirus (for a review, see Antivirals against AIDS (2000), M.Dekker, Inc.), such as:
- retroviral enzyme inhibitors, as detailed herein after,
- drugs effective against non-enzymatic retroviral proteins (glycosylated or not) which are involved in or essential for the replication of a retrovirus (e.g. HIV gp120), for instance the immunogen based on the gp120 envelope antigen discussed in *Nature* (2001) 410:966, and
- drugs that block a protein (e.g. a receptor or a cellular compound) that is involved in or essential for the entry of a retrovirus, e.g. CCR5 inhibitors (such as for instance the TAK-779 molecule disclosed by Dragic et al. in *Proc. Natl. Acad. Sci. USA* (2000) 97:5639-44.

Such biological properties or combination of properties makes them a most attractive pharmaceutically active ingredient namely in the so-called combination therapy of HIV infection in humans.

Therefore, in its broader meaning, the present invention relates to the use of a derivative of a pyrano[2,3-d:6,5-d'] dipyrimidine for the manufacture of a medicine for the treatment of a retroviral infection in retrovirus-infected patients, especially lentivirus-infected patients, and more particularly HIV-infected patients.

In another embodiment, the present invention is also based on the unexpected discovery of a (sub)synergistic effect (as hereinafter defined) against retroviral infection in human cell culture when used in combination with one or more anti-retroviral drugs such as reverse transcriptase inhibitors or protease inhibitors. The synergistic properties makes them most useful namely in the so-called combination therapy of HIV infection in humans.

It is most important to notice that International patent publication WO 99/25718 discussed herein-above does not suggest any activity of these compounds against HIV or any retroviral enzyme inhibition, and does not either suggest treating immunodeficiency itself, but only consequences thereof in immunodeficient patients.

### BRIEF DESCRIPTION OF THE DRAWINGS

Figure 1 represents the result of viral production in a time-of-addition experiment for a derivative of pyrano[2,3-d:6,5-d'] dipyrimidine used in this invention, as compared to other retroviral agents.
Figure 2 represents an isobologram plotting the fractional inhibitory concentrations of zidovudine and of a derivative of pyrano[2,3-d:6,5-d'] dipyrimidine used in this invention in the combined inhibitory action of these compounds on the cytopathic effect of HIV-1 induced in MT-4 cells.
Figure 3 represents an isobologram plotting the fractional inhibitory concentrations of nelfinavir and of a derivative of pyrano[2,3-d:6,5-d'] dipyrimidine used in this invention in the combined inhibitory action of these compounds on the cytopathic effect of HIV-1 induced in MT-4 cells.
Figure 4 represents an isobologram plotting the fractional inhibitory concentrations of nevirapine and of a derivative of pyrano[2,3-d:6,5-d'] dipyrimidine used in this invention in the combined inhibitory action of these compounds on the cytopathic effect of HIV-1 induced in MT-4 cells.
Figure 5 shows inhibition of HIV-1 integrase activity by means of a derivative of pyrano[2,3-d:6,5-d'] dipyrimidine used in this invention.
Figure 6 shows the results of a kinetic cleavage reaction in the absence or presence of a derivative of pyrano[2,3-d:6,5-d'] dipyrimidine used in this invention.

### DETAILED DESCRIPTION OF THE INVENTION

The present invention first relates to the use of a derivative of a pyrano[2,3-d:6,5-d'] dipyrimidine for the manufacture of a medicine for the treatment of a retroviral infection, especially a lentiviral infection, and more particularly a HIV-infection in a mammal.

In a second aspect, the present invention provides the use of a composition comprising (a) one or more derivatives of a pyrano[2,3-d:6,5-d'] dipyrimidine and (b) one or more anti-retroviral, including drugs effective against one or more retroviral or cellular proteins involved in the entry and/or replication of a retrovirus (e.g. retroviral enzyme inhibitors) as a medicine (or for the manufacture of a medicine), preferably against (or for the treatment of) a retroviral infection in a mammal and more preferably in respective proportions such as to provide a synergistic effect against (or in the treatment of) a retroviral infection in a mammal. In a third aspect, the present invention provides a product containing (a) one or more derivatives of a pyrano[2,3-d:6,5-d'] dipyrimidine, and (b) one or more anti-retroviral drugs, including drugs effective against one or more retroviral or cellular proteins involved in the entry and/or replication of a retrovirus (e.g. retroviral enzyme inhibitors), preferably in respective proportions such as to provide a synergistic effect against a retroviral infection in a mammal, as a combined preparation for simultaneous, separate or sequential use in retroviral infection therapy. In a fourth aspect, the present invention provides the use of an effective amount of a combined therapeutic preparation comprising (a) one or more derivatives of a pyrano[2,3-d:6,5-d'] dipyrimidine and (b) one or more anti-retroviral drugs, including drugs effective against one or more retroviral or cellular proteins involved in the entry and/or replication of a retrovirus (e.g. retroviral enzyme inhibitors) in respective proportions such as to provide a synergistic effect against retroviral infection for the manufacture of a medicament for the treatment of a retroviral infection in a mammal, comprising simultaneously, separately or sequentially administering to the mammal in need of such treatment.

In the various aspects of the present invention, the retroviral infection to be treated is preferably a lentiviral, and more preferably a HIV infection.

In the various aspects of the present invention, the pyrano[2,3-d:6,5-d'] dipyrimidine derivative preferably is a compound having the formula 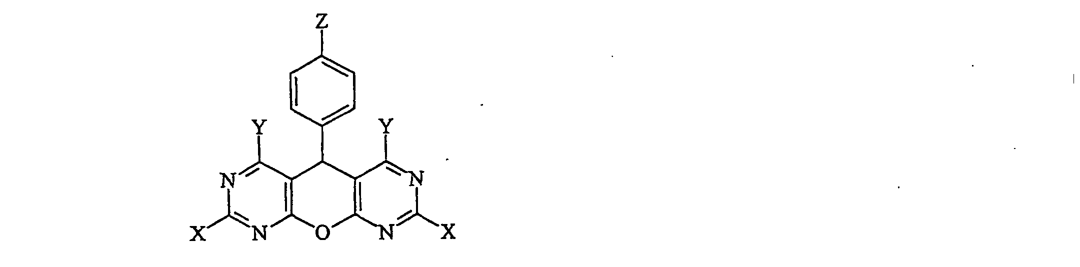 wherein :
- X is selected from hydroxy, halo and thiol,
- Y is selected from hydroxy, methoxy and halo, and
- Z is selected from hydrogen, halo and nitro,
a pharmaceutically acceptable addition salt or a stereochemically isomeric form thereof, and may be obtained for instance by reacting the pyridine salt of a 5,5' arylidene *bis*-barbituric acid (e.g. when both X and Y are hydroxy) or a 5,5'-arylidene bis (2-thio) barbituric acid (e.g. when X is a thiol and Y is hydroxy) with phosphorus oxychloride POCl₃, then with phosphorus oxide P₂ O₅, or alternatively (e.g. when X is hydroxy, Y is methoxy and Z is p-nitro) by reacting 6-methoxyuracil, p-nitrobenzaldehyde, acetic acid and acetic anhydride at a temperature near to the boiling temperature of acetic acid. Variants of these methods are disclosed in WO 99/25718. A representative member of this family of derivatives is 5-(4-nitrophenyl)-2,8-dithiol-4,6-dihydroxy-5*H*-pyrano[2,3-d:6,5-d']dipyrimidine. Interestingly it was observed that, within this family of derivatives, molecules with a sulfhydryl function at positions 2 and 8 tend to show more pronounced antiviral activity than the corresponding compounds with hydroxyl substituents at the same positions.

The pharmaceutically acceptable addition salts as mentioned hereinabove are meant to include the therapeutically active non-toxic acid addition salt forms which the compounds having the above formula are able to form and which may conveniently be obtained by treating the base form of such compounds with an appropriate acid. Examples of such appropriate acids include, for instance, inorganic acids such as hydrohalic acids, e.g. hydrochloric or hydrobromic acid, sulfuric acid, nitric acid, phosphoric acid and the like; or organic acids such as, for example, acetic, propanoic, hydroxyacetic, 2-hydroxypropanoic, 2-oxopropanoic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclohexanesulfamic, salicylic (i.e. 2-hydroxybenzoic), p-aminosalicylic, pamoic and the like. Conversely the salt form can be converted by treatment with an appropriate alkali into the free base form. The term " pharmaceutically acceptable addition salt " as used herein above includes the solvates which the compounds having the above formula, as well as their salts as above defined, are able to form, such as for example hydrates, alcoholates and the like.

The pyrano[2,3-d:6,5-d'] dipyrimidine derivatives used as a therapeutically active ingredient in the present invention should preferably be present in a substantially pure form, i.e. free from chemical impurities (such as co-products or residual solvents) resulting from their manufacturing and/or handling processes in view to safely control the therapeutic program for which they are intended. They may be present, when they possess at least an asymmetric carbon atom, either as a racemic mixture or in the form of a substantially pure stereoisomer or enantiomer of the said compound obtained from the racemic mixture by standard fractionation methods, including simulated moving bed technology.

The term "stereochemically isomeric forms" as used herein before defines all the possible isomeric forms which the compounds of formula (I) may possess. Unless otherwise stated, the chemical designation of compounds denotes the mixture of all possible stereochemically isomeric forms, said mixtures containing all diastereomers and enantiomers of the basic molecular structure. More particularly, stereogenic centers may have either the R- or S-configuration. Pure stereoisomeric forms of the said compounds are defined as isomers substantially free of other enantiomeric or diastereomeric forms of the same basic molecular structure. In particular, the term "stereoisomerically pure" or "chirally pure" relates to compounds having a stereoisomeric excess of at least 80% (i.e. at least 90% of one isomer and at most 10% of the other possible isomers), preferably at least 90%, more preferably at least 94% and most preferably at least 97%. The terms "enantiomerically pure" and "diastereomerically pure" should be understood in a similar way, having regard to the enantiomeric excess, respectively the diastereomeric excess, of the mixture in question.

Consequently, if a mixture of enantiomers is obtained during the preparation method, it can be separated by liquid chromatography using a suitable chiral stationary phase. Suitable chiral stationary phases are, for example, polysaccharides, in particular cellulose or amylose derivatives. Commercially available polysaccharide based chiral stationary phases are ChiralCel™ CA, OA, OB, OC, OD, OF, OG, OJ and OK, and Chiralpak™ AD, AS, OP(+) and OT(+). Appropriate eluents or mobile phases for use in combination with said polysaccharide chiral stationary phases are hexane and the like, modified with an alcohol such as ethanol, isopropanol and the like.

The term "substantially pure " as used herein before means a chemical purity, as determined by methods conventional in the art such as high performance liquid chromatography, of at least about 96%, preferably at least 98% and more preferably at least 99%.

In the various aspects of the present invention, the retroviral enzyme inhibitors used as drugs (b) may belong to categories already known in the art and include, among others:
- HIV-1 IN inhibitors such as reviewed hereinabove in this specification,
- nucleoside reverse transcriptase inhibitors such as for instance zidovudine, lamivudine, didanosine, stavudine, zalcitabine and the like,
- non-nucleoside reverse transcriptase inhibitors such as for instance nevirapine, delavirdine and the like,
- other reverse transcriptase inhibitors such as for instance foscarnet sodium and the like,
- HIV-1 protease inhibitors such as for instance saquinavir, ritonavir, indinavir, nelfinavir and the like.

The product provided in the third aspect of the present invention may be in the form of a pharmaceutical preparation or composition wherein at least one of (a) and (b) is in admixture with at least a pharmaceutically acceptable carrier. The term "pharmaceutically acceptable carrier" as used herein means any material or substance with which the composition of active ingredients (a) and (b) is formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, pellets or powders.

Suitable pharmaceutical carriers for use in the said pharmaceutical compositions and their formulation are well known to those skilled in the art, and there is no particular restriction to their selection within the present invention. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-steps procedure, with the selected carrier material and, where appropriate, the other additives such as surface-active agents. may also be prepared by micronisation, for instance in view to obtain them in the form of microspheres usually having a diameter of about 1 to 10 µm, namely for the manufacture of microcapsules for controlled or sustained release of the active ingredients.

Suitable surface-active agents to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids (C₁₀-C₂₂), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanylphosphatidyl-choline, dipalmitoylphoshatidyl -choline and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenol -polyethoxyethanol, castor oil polyglycolic ethers, polypropylene/polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having 4 hydrocarbon radicals optionally substituted with halo, phenyl, substituted phenyl or hydroxy; for instance quaternary ammonium salts containing as N-substituent at least one C₈-C₂₂ alkyl radical (e.g. cetyl, lauryl, palmityl, myristyl, oleyl and the like) and, as further substituents, unsubstituted or halogenated lower alkyl, benzyl and/or hydroxy-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbuch", 2^{nd} ed. (Hanser Verlag, Vienna, 1981) and "Encyclopaedia of Surfactants (Chemical Publishing Co., New York, 1981).

Additional ingredients may be included in order to control the duration of action of the active ingredient in the composition. Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition may require protective coatings.

Pharmaceutical forms suitable for injectionable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol and the like and mixtures thereof.

In view of the fact that ingredients (a) and (b) do not necessarily bring out their joint therapeutic effect directly at the same time in the mammal to be treated, the product provided in the third aspect of the present invention may also be in the form of a medical kit or package containing the two ingredients in separate but adjacent form. In the latter context, each of ingredients (a) and (b) may therefore be formulated in a way suitable for an administration route different from that of the other ingredient, e.g. one of them may be in the form of an oral or parenteral formulation whereas the other is in the form of an ampoule for intravenous injection or an aerosol.

According to the product being the third aspect of the present invention:
- the active ingredients (a) and (b) may be administered to the mammal (including a human) to be treated by any means well known in the art, i.e. orally, intranasally, subcutaneously, intramuscularly, intradermally, intravenously, intra-arterially, parenterally or by catheterization.
- the effective amount of the combined preparation of (a) and (b) preferably is an anti-retroviral amount, e.g. a retroviral enzyme inhibiting amount. More preferably, it is an integrase inhibiting amount of derivative (a) and a retroviral enzyme inhibiting amount of drug (b). Still more preferably when the said retroviral enzyme inhibitor drug (b) is a protease inhibitor, its effective amount is a protease inhibiting amount. When the said retroviral enzyme inhibitor drug (b) is a reverse transcriptase inhibitor, its effective amount is a reverse transcriptase inhibiting amount. When the said retroviral enzyme inhibitor drug (b) is an integrase inhibitor other than a derivative of a pyrano[2,3-d:6,5-d'] dipyrimidine, its effective amount is an integrase inhibiting amount. When (b) is a drug effective against a non-enzymatic retroviral protein (glycosylated or not) involved in the replication of a retrovirus (e.g. the immunogen based on the HIV gp120 envelope antigen), it should also be used in its usual effective amount. When (b) is a CCR5 inhibitor, it should be used in a CCR5 inhibiting amount.
- ingredients (a) and (b) may be administered simultaneously but it may also be beneficial to administer them separately or sequentially, for instance within a relatively short period of time (e.g. within about 24 hours) in order to achieve their functional fusion in the body to be treated.

The following examples are provided for illustrative purposes only and should in no way be interpreted as limiting the scope of the present invention.

### EXAMPLES

For the performance of the working examples of this invention, the following compounds and experimental procedures were used.

Pyrano[2,3-d:6,5-d'] dipyrimidine derivatives were obtained according to the method disclosed in WO 99/25718. AZT was synthesized as previously described by Horwitz et al. in *J.Org. Chem.* (1964) 29:2076-8. Tivirapine and loviride were obtained from Janssen Research Foundation (Beerse, Belgium). Nevirapine was obtained from Boehringer Ingelheim (Ridgefield, CN). Ritonavir was provided by Abbott laboratories (Abbott Park, Illinois). Dextran sulfate with a molecular weight of 5,000 was purchased from Sigma. Nelfinavir was obtained from Agouron Pharmaceuticals (La Jolla, California). Bicyclam AMD3100 was obtained from Amormed.

MT-4 (disclosed by Miyochi et al. in *Gann. Monogram.* (1982) 28:219-228) and C8166 (disclosed by Salahuddin et al. in *Virology* (1983) 129:51-64) cells were grown in a humidified atmosphere with 5% CO₂ and maintained in RPMI 1640 medium supplemented with 10% heat inactivated fetal calf serum, 2 mM L-glutamine, 0.1 % sodium bicarbonate and 20 µg/ml of gentamicin.

HIV-1 (III_{B}) virus was described by Popovic et al. in *Science* (1984) 224:497-500. HIV-1 (NDK) was also described by Spire et al. in *Gene* (1989) 81:275-284. HIV-1 (hereinafter referred as NL 4.3 and disclosed by Adachi et al. in *J.Virol.* (1986) 59:284-291) is a molecular clone obtained from the National Institute of Health (Bethesda, Maryland). The strains 13MB1 (L1001) and 39MN1 (Y181C) were isolated in our laboratory after serial passage of HIV-1 (III_{B}) and HIV-1 (NDK) in MT-4 cells in the presence of tivirapine or loviride respectively. L1, L2, L4 and L6 are clinical isolates from a single seropositive patient before and after sequential treatment with the dideoxynucleosides (ddN) analogues zidovudine, didanosine, zalcitabine, stavudine and lamivudine and the non-nucleoside reverse transcriptase inhibitor loviride. According to Schmidt et al. (cited *supra),* the strain L6 contains the following mutations in reverse transcriptase: V751, F77L, K103N, F116Y, Q151M and M184V. HIV-1 or HIV-2 (ROD) (disclosed by Barré-Sinoussi et al. in *Science* (1983) 220:868-871) and HIV-2 (EHO) (disclosed by Rey et al. in *Virology* (1989) 173:258-267) stocks were obtained from the culture supernatant of HIV-1- or HIV-2-infected cell lines according to Pauwels et al. in *J*. *Virol. Methods* (1987) 16:171-185 and Schols et al. in *J.AIDS* (1989) 2:10-15. Simian immunodeficiency virus [ SIV(MAC251)] was obtained from (Smith Kline, Rixensart, Belgium), stocks being prepared from the supernatant of SIV-infected MT-4 cells.

### EXAMPLE 1 - inhibitory activity of pyrano[2,3-d:6,5-d'] dipyrimidine derivatives against the replication of HIV or SIV in a cell culture model for acute intection.

The inhibitory activity of 8 different compounds (hereinafter noted A to H) in a series of 5-(4-substituted-phenyl)-5*H*-pyrano[2,3-d:6,5-d']dipyrimidines having the formula: 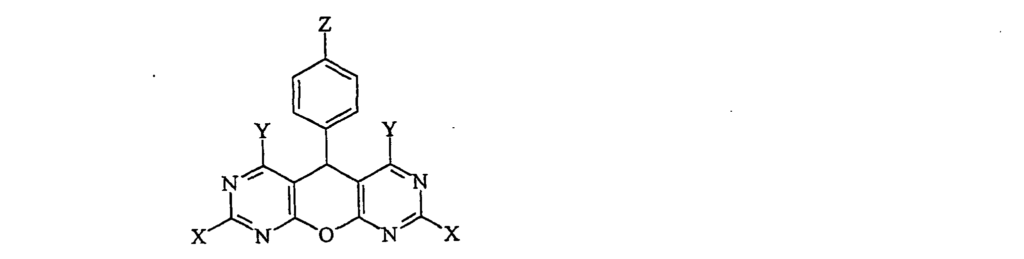 wherein X, Y and Z are as defined in the following table 1 was tested for their potential to inhibit the replication of HIV and SIV in a cell culture model for acute infection.

**Table 1**

| compound | X | Y | Z |
|---|---|---|---|
| A | OH | OH | I |
| B | Cl | Cl | H |
| C | OH | OH | H |
| D | OH | OCH₃ | NO₂ |
| E | OH | OH | Br |
| F | OH | OH | Cl |
| G | SH | OH | Cl |
| H | SH | OH | NO₂ |

Selected compounds were tested against HIV-1(III_{B}), two HIV-2 strains (ROD and EHO), and SIV (MAC251) for inhibition of virus-induced cytopathicity in MT-4 cells, using the colorimetric test described by Pauwels et al. in *J. Virol. Methods* (1988) 20:309-321, evaluation being made 5 days post-infection.

Cytotoxicity of these compounds was determined in parallel by measuring the viability of mock-infected MT-4 or C8166 cells 5 days post-infection. The antiviral activity and cytotoxicity data, including average values and standard deviations for at least two separate experiments, are depicted in Table 2, wherein:
- EC₅₀ represents the 50% effective concentration, i.e. the concentration required to inhibit the viral cytopathic effect by 50% in cell culture, and
- CC₅₀ represents the 50% cytotoxic concentration, i.e. the concentration that reduces by 50% the viability of MT-4 cells.

**Table 2**

| EC₅₀ (µM) | | | | | | |
|---|---|---|---|---|---|---|
| compound | HIV-1 | | HIV-2 | | SIV | CC₅₀ (µM) |
| | III_{B} | L1 | ROD | EHO | MAC251 | |
| A | >276 | >276 | >276 | >276 | >276 | >276 |
| B | >17.5 | >17.5 | >17.5 | >17.5 | >17.5 | 17.5 |
| C | 170±45 | 116±30 | >383 | 169±27 | 194.2 | >383 |
| D | >313 | >313 | >313 | >313 | >313 | >313 |
| E | 273±38 | >309 | >309 | >309 | >309 | >309 |
| F | >347 | >347 | >347 | >347 | >347 | >347 |
| G | 56.8±30 | 101±32 | >137 | >137 | 100.4 | 137 |
| H | 8.9±0.7 | 3.7±1.8 | 30±6.2 | 3.7±0.1 | 5.5±0.9 | 120.6 |
| nevirapine | 0.04 | 0.06±0.01 | >97.6 | >97.6 | >97.6 | >97.6 |

Derivatives C, E, G and H were active against the replication of HIV-1(III_{B)}. The most active compound of this series, H or 5-(4-nitrophenyl)-2,8-dithiol-4,6-dihydroxy-5*H*-pyrano[2,3-d:6,5-d']dipyrimidine (named V-165 on all attached figures), was active at a 50% effective concentration (EC₅₀) of 11.4 µM. H was also active against HIV-1 (NDK, NL4.3 and L1), HIV-2 (ROD and EHO) and SIV (MAC251) at EC₅₀ values ranging from 5.5 to 30 µM. In contrast, the non-nucleoside reverse transcriptase inhibitor nevirapine, tested by way of comparison, was inactive against the replication of HIV-2 (ROD and EHO) and SIV (MAC251) at 98 µM in MT-4 cells.

### EXAMPLE 2 - inhibitory activity of certain pyrano[2,3-d:6,5-d'] dipyrimidine derivatives against drug-resistant HIV-1 strains.

The antiviral activity of compounds G and H was tested, using the same method as in example 1, against various drug-resistant HIV-1 strains. Results are indicated in the following table 3, wherein EC₅₀ has the same meaning as in example 1 and the fold increase in EC₅₀ is by comparison to the antiviral activity against the wild type (WT) strain. Compound H was active against HIV-1 strains that are resistant to the viral entry antagonists dextran sulfate or bicyclam. HIV-1 strains selected for resistance against the non-nucleoside reverse transcriptase inhibitors (NNRTIs) tivirapine or loviride were inhibited by both compounds.

**Table 3**

| | | | EC₅₀ (µM) fold increase | |
|---|---|---|---|---|
| Mutant strain | WT strain | Compound used to select resistance | G | H |
| Resistant to viral entry inhibitors | | | | |
| NL43/DS5000 | NL4.3 | dextran | - | 19.4±1.4(1.3) |
| | | sulfate | | |
| NL43/AMD3100 | NL4.3 | bicyclam | - | 19.1±1.4(1.3) |

| Resistant to RT inhibitors | | | | |
|---|---|---|---|---|
| L2 | L1 | NRTIs | 95.6±6.5 (0.9) | 6.0±2.3 (1.6) |
| L4 | L1 | NRTIs | 163.3±3.7 (1.6) | 11.7±0.7 (3.2) |
| L6 | L1 | NRTIs+Iovirid | 183.2±101.5 (1.8) | 10.5±5.1 (2.8) |
| 13MB1 | III_{B} | Tivirapine | 134.3±10.5 (2.4) | 8.7±3.5 (1.0) |
| 39MN1 | NDK | loviride | 161.6±66.4 (2.5) | 41.6±3.0 (2.2) |

Additionally, we observed a maximal 3-fold reduction in inhibitory potency of compound H against the three multi-drug resistant (MddNR) HIV-1 strains L2, L4 and L6, taking into account that the parental strain L1 was more susceptible (EC₅₀: 3.7±1.8 µM) to compound H than III_{B}.

### EXAMPLE 3 - Combined inhibitory effects of 5-(4-nitrophenyl)-2,8-dithiol-4,6-dihydroxy-5H-pyrano[2,3-d:6,5-d']dipyrimidine with various HIV-1 inhibitors.

The antiviral effect of compound H was tested in combination with the nucleoside reverse transcriptase inhibitor zidovudine, the non-nucleoside reverse transcriptase inhibitor nevirapine and the protease inhibitor nelfinavir.

The combined inhibitory effect on HIV-1-induced cytopathic effect was evaluated by the isobologram method, as previously described by Elion et al. in *J. Biol. Chem*. (1954) 208:477-488 and by Baba et al. in *Antimicrob. Agents Chemother.* (1984) 25:515-517.

EC₅₀, as defined herein before, was used for calculating the fractional inhibitory concentration (hereinafter referred as FIC). When the minimum FIC index corresponding to the FIC of combined compounds (e.g., FICₓ + FIC_{y}) is equal to 1.0, the combination is said to be additive; when it is beween 1.0 and 0.5, the combination is by definition said to be subsynergistic, and when it is <0.5, the combination is by definition said to be synergistic. When the minimum FIC index is between 1.0 and 2.0, the combination is said to be subantagonistic and, when it is >2.0, the combination is defined as antagonistic.

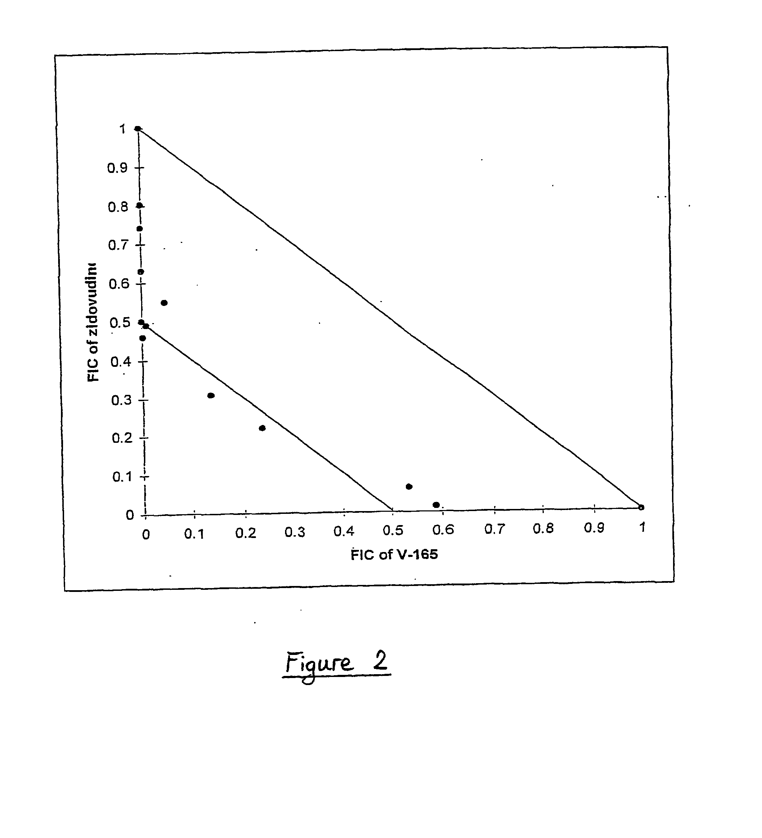

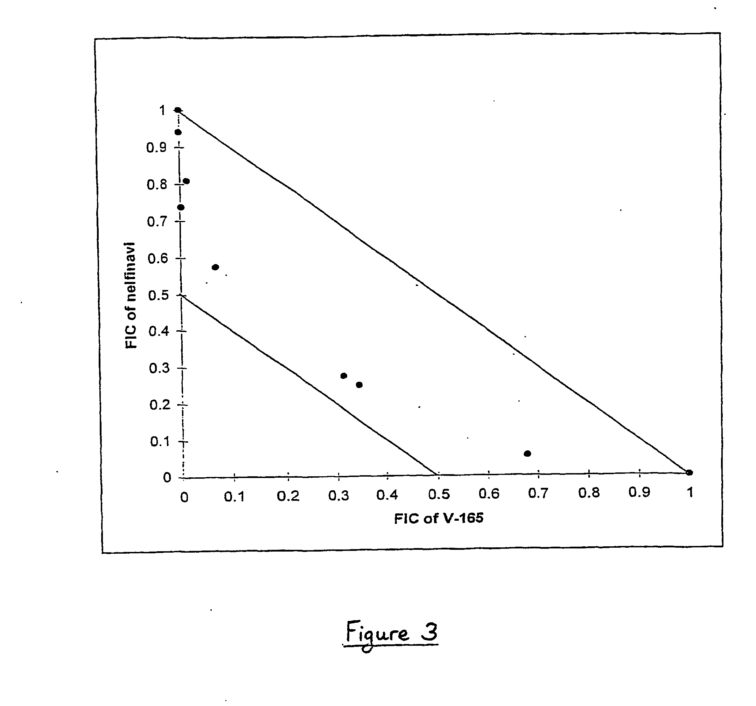

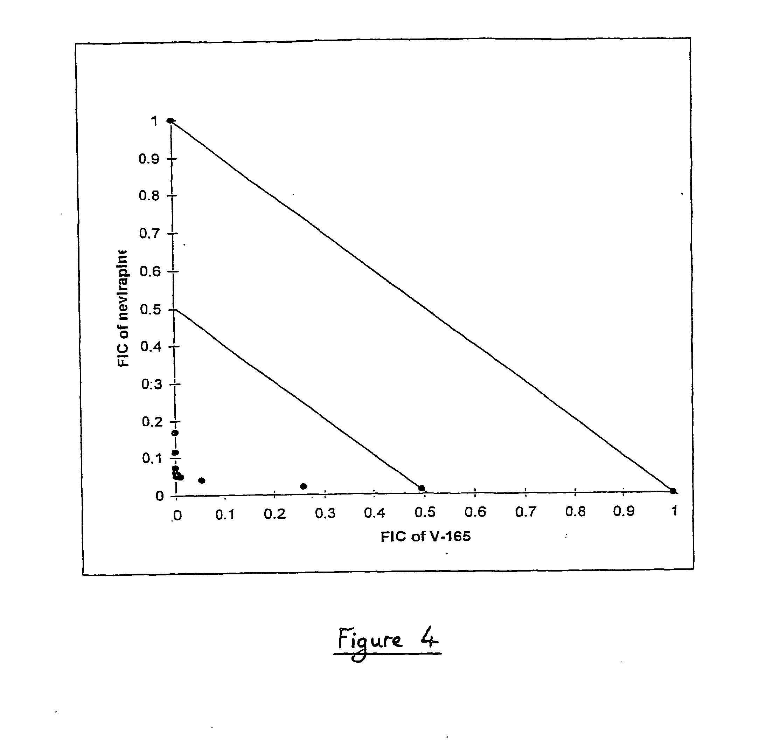

The results of these tests are reported in the isobolograms provided in figures 2 to 4, wherein lines represent FIC equal to 1.0 and 0.5 respectively, which show that the added values FIC_{zidovudine} + FIC_{V-165} and FIC_{nelfinavir} + FIC_{V-}-₁₆₅fell between 0.5 and 1 and the added values FICₙₑᵥᵢᵣₐₚᵢₙₑ + FIC_{V-165} fell below 0.5.

### Example 4 - time of intervention of 5-(4-nitrophenyl)-2,8-dithiol-4,6-dihydroxy-5H-pyrano[2,3-d:6,5-d']dipyrimidine in the replicative cycle.

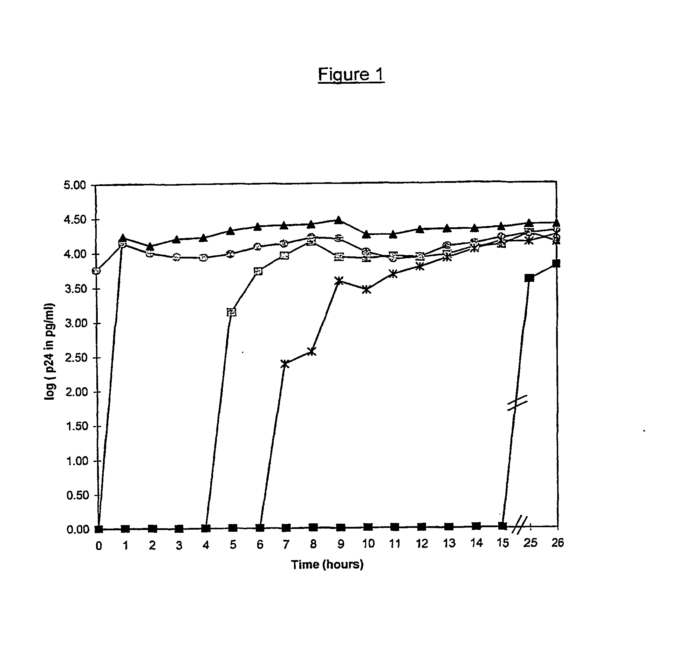

A time of addition experiment was carried out in order to investigate which step of the replicative cycle was inhibited by compound H. Briefly, this experiment determines how long the addition of an anti-HIV compound can be postponed before losing its antiviral activity in the viral replication cycle. MT-4 cells were infected with HIV-1(III_{B)} at a multiplicity of infection (m.o.i.) of 0.5 in order to synchronize all steps of viral replication and the various antiviral test compounds were added at different times (ranging from 0 up to 26 hours) after infection. Viral p24 antigen production was determined 31 hours post-infection (using HIV-1 p24 Core Profile ELISA, Du Pont, Dreieich, Germany) and is expressed as the log₁₀ of the p24 Ag content in pg/ml. Reference compounds with a known mode of action were included in this experiment. Dextran sulfate, a polyanion, interferes with binding of the virus to the cell. The nucleoside analogue AZT inhibits the reverse transcription process. Ritonavir is a proteolytic cleavage inhibitor. The reference compounds (dextran sulfate, AZT and ritonavir) were added at a standardized concentration corresponding to 100 times the EC₅₀ value, determined at a m.o.i. of 0.01. Compound H was added at 250 µM. In figure 1 showing the results of this experiment, the following symbols were used: (•) for control, (▲) for dextran sulfate (20 µM), (■) for AZT (1.9 µM), (■) for ritonavir (2.8 µM) and (*) for compound H (250 µM).

According to figure 1, addition of compound H can be postponed for 6 hours after virus infection, suggesting an interaction at the moment of integration (DNA strand transfer) since a known diketo acid integrase inhibitor tested in a similar time-of-addition experiment lost its activity when added more than 6 hours beyond infection. However this experiment cannot exclude that compound H would interact with reverse transcription as well, since the experiment only reveals the last step targeted during viral replication.

### EXAMPLE 5 - inhibition of lentiviral vector-mediated transduction by 5-(4-nitrophenyl)-2,8-dithiol-4,6-dihydroxy-5H-pyrano[2,3-d:6,5-d']dipyrimidine.

In order to corroborate the interference of the inhibitor with the HIV reverse transcription and/or integration steps, single round infections of 293T cells were done using HIV-1-derived vectors pseudotyped with the VSV-G envelope and carrying luciferase as a reporter gene, according to the method described by Naldini et al. in *Science* (1996) 272:263-7. Specifically, HIV-1-derived vector particles, pseudotyped with the envelope of vesicular stomatitis virus (VSV), were produced by transfecting 293T cells with three plasmids: a packaging plasmid pCMVΔR8.2 or pCMVΔR8.91, a plasmid encoding the envelope of vesicular stomatitis virus (pMDG) and a plasmid encoding a reporter gene flanked by two long terminal repeats (LTRs) (pHR'-CMVluciferase). All plasmids were obtainable through the European Gene Vector Database and Repository and were provided by Genethon III, CNRS URA 1923, Evry (france) and by the University of Geneva, Department of Genetics and Microbiology (Switzerland). For transfection of a 10 cm dish of 293T cells, a 700 µl mixture of three plasmids was made in 150 mM NaCl (20 µg of vector plasmid, 10 µg of packaging construct and 5 µg of envelope plasmid). To this DNA solution 700 µl of polyethyleneimine (PEI) (available from Aldrich) (110 µl of a 10 mM stock solution in 150 mM NaCl) was added slowly. After 15 minutes at room temperature, the DNA-PEI complex was added dropwise to the 293T cells in a DMEM medium supplemented with 1% fetal calf serum (FCS). After overnight incubation, the medium was replaced with a medium containing 10% FCS. Supernatants were collected from day two to five post-transfection. The vector particles were sedimented by ultracentrifugation in a swinging-bucket rotor (SW27 Beckman, Palo Alto, California) at 25,000 rpm for two hours at 4°C. Pellets were redissolved in PBS resulting in a 100-fold concentration. Different viral stocks were normalized based on p24 antigen content (HIV-1 p24 Core Profile ELISA from Du Pont, Dreieich, Germany). Inhibitors were evaluated in 96-well plates. For each well, 2,000 pg of vector and 2 µg/ml polybrene were added to a confluent monolayer of 293T cells grown in 100 µl of DMEM/1% FCS. Inhibitors at different concentrations were included. 48 hours after transfection, cells were lysed and luciferase activity was measured in the Lumicount™ (Packard, Meriden, Connecticut) using the Luciferase Assay System™ (Promega Benelux, Leiden, Netherlands). Normalization for cell count was done by determining protein concentration using BCA™ Protein Assay Reagent (Pierce, Illinois).

In this assay a decrease in luciferase activity is a measure for the inhibition of the early HIV replication steps. Inhibition of transduction was obtained with compound H at an average EC₅₀ value of 17 µM. The control reverse transcriptase inhibitor AZT inhibited lentiviral transduction at an EC₅₀ value of 49 nM. The lack of gp120 in the viral particle excludes HIV-type viral entry as a target. Compound H also inhibited the transduction by a second generation vector lacking the accessory factors nef, vif, vpr and vpu (such as described by Zufferey et al. in *Nature Biotechnol*. (1997) 15:871-5) to the same extent, thus excluding a major role for these HIV proteins in the antiviral mechanism. These assays corroborate HIV-1 reverse transcription and/or integration as the targets for the antiviral effect of the compound of the invention in cell culture.

### EXAMPLE 6 - inhibition of HIV reverse transcriptase activity by certain pyrano[2,3-d:6,5-d'] dipyrimidine derivatives.

Inhibition of HIV-1 reverse transcriptase enzymatic activity was evaluated for some compounds of the invention and some reference compounds while using the procedures for obtain virion-derived enzyme and to assay for reverse transcriptase activity as described by Debyser et al. in *Proc*. *Natl. Acad. Sci.* USA (1991) 88:1451-5. Poly(rC).oligo(dG) and poly (rA).oligo(dT) were used as template-primers, and 8-[³H]-dGTP and [³H]-dTTP were used as radiolabelled substrates. The final 8-[³H]-dGTP and [³H]-dTTP concentrations in the reaction mixture were 2.5 µM. Recombinant HIV-1 RT (HXB2) was obtained as described by Jonckheere et al. in *J. Virol. Methods* (1996) 61:113-125. Results of these experiments are shown in table 4 below, wherein IC₅₀ is the 50% inhibitory concentration, i.e. the concentration required to inhibit recombinant HIV-1 RT activity by 50% using poly(C).oligo(dG) or poly(A).oligo(dT) as template-primers.

**Table 4**

| Compound | IC₅₀ (µM) | |
|---|---|---|
| | poly(C).oligo(dG) | Poly(A).oligo(dT) |
| C | 15.1 ± 5.9 | 33.1 ± 15.1 |
| E | >494 | >494 |
| G | 25.8 ± 15.9 | 35.2 ± 0.4 |
| H | 4.8 ± 2.2 | 24.6 ± 7.2 |
| Tivirapine | 0.3 ± 0.2 | 0.7 ± 0.2 |
| Dextran | 7.3 ±3.2 | 22.0 ± 8.8 |
| sulfate | | |

### EXAMPLE 7 - inhibition of HIV integrase activity by certain pyrano[2,3-d:6,5-d'] dipyrimidine derivatives.

Inhibition of 3'-processing, DNA strand transfer and the over-all integration reaction were measured in oligonucleotide-based assays, as described by Cherepanov et al. in *Mol. Pharmacol.* (1997) 52:771-780 using the following methodology.

First, recombinant His-tagged HIV-1 integrase was produced from the plasmid pRP1012 (available from Netherlands Cancer Institute, Amsterdam), encoding HIV-1 IN (strain HTLV III), in *E. coli* PC1 (BL21(DE3)(pLysS)Δ *endA*::Tc^{R}) and purified on a Ni-nitrilotriacetic acid column (Qiagen, Hilden, Germany), followed by a HighTrap-heparin column (Pharmacia) as described by Cherepanov et al. (cited *supra).*

The following high performance liquid chromatography-purified deoxyoligonucleotides were purchased from Amersham-Pharmacia Biotech:

INT1, 5'-TGTGGAAAATCTCTAGCAGT;

INT2, 5'-ACTGCTAGAGATTTTCCACA;

T35, 5'-ACTATACCAGACAATAATTGTCTGGCCTGTACCGT;

SK70, 5'-ACGGTACAGGCCAGACAATTATTGTCTGGTATAGT;

The oligonucleotides INT1 and INT2 correspond to the U5 end of the HIV-1 LTR. The oligonucleotide INT1 was purified through a 20% denaturing polyacrylamide/urea gel and was 5'-end labeled using polynucleotide T4 kinase and [□-³²P]ATP (available from Amersham-Pharmacia Biotech). The DNA substrate for the integrase reactions was made by annealing INT1 and INT2. An equimolar mixture of the two oligonucleotides in the presence of 100 mM NaCl was heated shortly at 95°C and allowed to cool slowly to room temperature. Likewise, annealing of SK70 and T35 resulted in a 35-bp dsDNA molecule that was used as a target DNA molecule (T35/SK70).

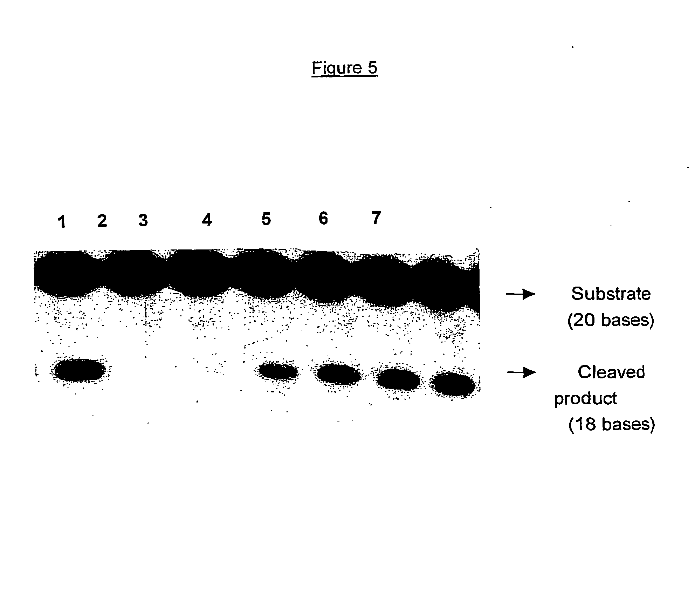

The final reaction mixture for the 3'-processing assay contained 20 mM 4-(2-hydroxyethyl)-1-piperazinethanesulfonic acid (hereinafter referred as HEPES), pH 7.5, 5 mM dithiothreitol, 10 mM MgCl₂, 75 mM NaCl, 15% (volume/volume) of a polyethylene glycol with a molecular weight of 8,000, 30 nM of the oligonucleotide substrate and 230 nM of the His-tag integrase in a volume of 10 µl. Reactions were started by the addition of the enzyme. Inhibitors were incubated shortly with the reaction components before the addition of integrase. Reactions were allowed to proceed at 37°C for 7 minutes and stopped by the addition of formamide loading buffer (95% formamide, 30 mM ethylenediamine tetraacetic acid, 0.1 % xylene cyanol, 0.1 % bromophenol blue, 0.1 % sodium dilaurylsulfate SDS). In the overall integration assay, this reaction was allowed to proceed for 60 minutes before the addition of a formamide dye. Figure 5 represents the result of the 3'-processing reaction carried out in the absence (lane 1) or in the presence of decreasing amounts of compound H (lanes 2 to 7). The concentrations of the inhibitor were 25 µM (lane 2), 5 µM (lane 3), 1 µM (lane 4), 0.2 µM (lane 5), 0.04 µM (lane 6) and 0.008 µM (lane 7).

Strand transfer was assayed in the following way: 30 nM DNA substrate was preincubated with 230 nM integrase at 37°C for 5 minutes in order to allow the cleavage reaction to occur. The composition of the reaction mixture was identical to that in the 3'-processing assay. After 5 minutes, 1 µl of excess target DNA (final concentration 250 nM) with or without inhibitor was added, and the samples were incubated at 37°C for one hour. This excess target DNA blocks competitively further binding of integrase to the viral substrate.

Reactions were stopped by the addition of formamide dye, and products were separated in a 15% denaturing polyacrylamide/urea gel. Autoradiography was performed by exposing the wet gel to X-ray film (CURIX RP1, available from Agfa-Gevaert, Belgium). Quantification of the results was performed using a Phosphorlmager device (Molecular Dynamics, Sunnyvale, California).

Results of these experiments are shown in table 5 below, providing IC₅₀ (µM) values as defined hereinabove.

**Table 5**

| Compound | 3'-processing | Overall integration | Strand-transfer |
|---|---|---|---|
| A | > 206.6 | > 206.6 | - |
| B | 74.0 ± 38.5 | 25.0 ± 4.5 | - |
| C | 22.4 ± 6.4 | 34.6 ± 11.3 | 782 ± 242 |
| D | > 250 | > 250 | - |
| E | 208.0 ± 82.2 | 298.3 ± 7.7 | > 1235 |
| F | 143.7 ± 81.3 | 97.1 ± 93.8 | - |
| G | 0.9 ± 0.5 | 1.0 ± 0.4 | 201.3 ± 119.7 |
| H | 0.9 ± 0.4 | 0.3 ± 0.2 | 16.1 ± 10.7 |

Compounds C, E, G and H inhibited the 3-processing and the overall integration reaction. Compound H also has a strong inhibitory effect on the DNA strand transfer reaction. A strong correlation between the antiretroviral activity observed in cell culture and the inhibitory activity in the integrase assays was observed. The best correlation was obtained between the inhibitory potency in cell culture and in the strand transfer assay (r² = 0.998).

### EXAMPLE 8 - kinetic cleavage assay of 5-(4-nitrophenyl)-2,8-dithiol-4,6-dihydroxy-5H-pyrano[2,3-d:6,5-d']dipyrimidine.

Preincubation of the 20 bp oligonucleotide (30 nM) with 230 nM integrase for 3 minutes resulted in the formation of the initial stable complex (ISC). The composition of the reaction mixture was identical to that of the 3'-processing reaction of example 7. When dextran sulfate with a molecular weight of 5,000 was added at a final concentration of 0.3 µM, further binding of integrase to the oligonucleotide was blocked. At several periods of time, 5 µl aliquots were taken and the reaction was stopped using 5 µl formamide loading buffer (having the same composition as above). The rate constant for the conversion of ISC into the cleaved product can be calculated using the formula: A-Be^{-kt}. In order to examine the inhibitory effect of compound H on 3'-processing, we repeated the same experiment in the presence of 50 µM compound (added after preincubation, together with dextran sulfate) and compared the rate constants: In the absence of compound H, it was calculated at 0.152 ± 0.038 min⁻¹, in its presence at 0.147 ± 0.025 min⁻¹. This quite similar value rules out that compound H directly affects the catalysis of 3'-processing.

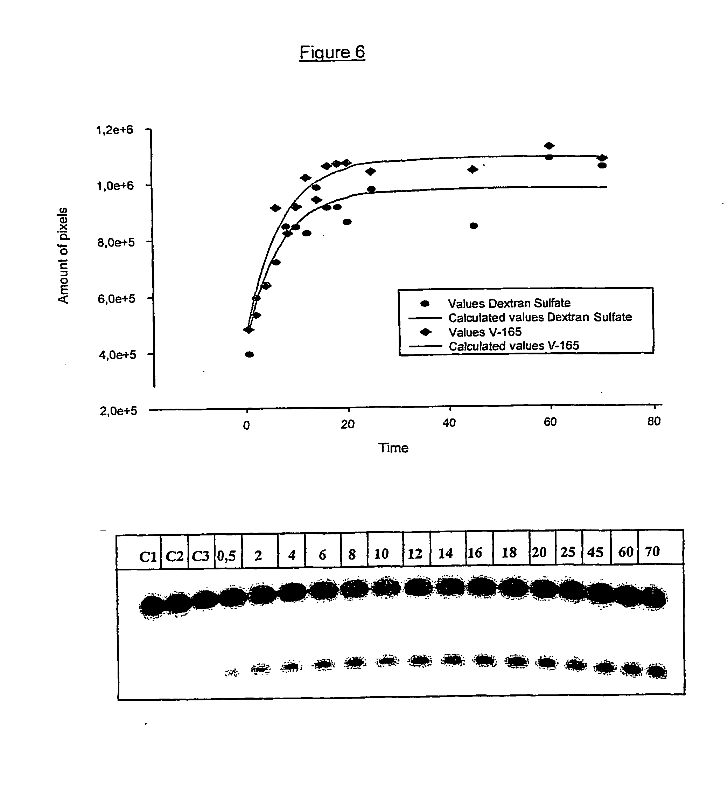

Additional results of this experiment are represented in figure 6 showing:
- in the upper panel, the kinetics of formation of the cleaved product in the absence (•) or presence (◆) of 50 µM of compound H, and
- in the lower panel, the denaturing gel after 0.5 to 70 minutes, including controls C1 (no enzyme added), C2 (dextran sulfate 5,000 added prior to preincubation) and C3 (no incubation).

### EXAMPLE 9 - HIV-1 integrase binding assay of 5-(4-nitrophenyl)-2,8-dithiol-4,6-dihydroxy-5H-pyrano[2,3-d:6,5-d']dipyrimidine.

Binding experiments were carried out using biosensor technology on BlAcore2000 (Biacore) and sensor chips SA™ with pre-immobilized streptavidin. The binding experiment was carried out at 37°C following the procedures of the manufacturer. The binding buffer B (20 mM HEPES at pH 7.5) contained 50 mM NaCl, 10 mM MgCl₂ and 5 mM DTT. First, the 3'-biotinylated oligonucleotide 5'-ACTGCTAGAGATTTTCCACACTGACTAAAAGGGTCAAAA-3' was bound to the sensor chip. Subsequently, the complementary 35-mer 5'-GACCCTTTTAGTCAGTGTGGAAAATCTCTAGCAGT-3' was hybridized to the captured oligonucleotide, resulting in an integrase recognition sequence at the free end. HIV-1 integrase (100µl of a 33 µM solution) diluted 10-fold in dilution buffer (10 mM Tris.HCl, pH7.5/750 mM NaCl/10% glycerol/l mM p-mercaptoethanol) and another 10-fold in buffer B, was injected at a final concentration of 330 nM with a flow rate of 10 µl/minute passing first a blank channel and then the channel with the specific oligonucleotide. A specific adsorption in the blank channel was subtracted. After preincubation of integrase with 93 µM of compound H, the binding of integrase to the DNA was completely abolished.

### EXAMPLE 10 - absence of intercalation of 5-(4-nitrophenyl)-2,8-dithiol-4,6-dihydroxy-5H-pyrano[2,3-d:6,5-d']dipyrimidine into double stranded DNA.

We verified whether compound H could be intercalated in double-stranded DNA, since it is known that intercalators stabilise the double helix by insertion between two consecutive base pairs. This stabilization is reflected by an increase of the melting temperature of the double strand (defined as the temperature where 50% of the double strand is dissociated in two single strands). We used the double-stranded DNA from the *in vitro* integration assay (INT1-INT2) at a fixed concentration of 1 µM for each oligodeoxynucleotide. A serial dilution of compound H (25, 5, 1, 0.2, 0.04 and 0 µM) was added to different cuvettes containing INT1-INT2, the temperature of which was first gradually increased (0.2°C/minute) from 15°C to 80°C and then decreased at the same rate down to 15°C, while monitoring the absorbance at 260 nm. Melting curves were determined with a Varian CARY 300 Bio Spectrophotometer. Cuvettes were thermostated with water circulating through the cuvette holder and the temperature of the solution was measured with a thermistor directly immersed in the cuvette. Temperature control and data acquisition were done automatically with a Compaq Deskpro computer. The samples were first heated and then cooled down at a rate of 0.2 °C per minute. Melting curves were evaluated taking the first derivative with the inflection point indicating the Tm. The variability was less than 0.5°C

The melting points thus determined remained unaffected (at 59°C) in the presence of various concentrations of compound H. This is a clear indication that compound H does not stabilize double-strand DNA by intercalation.

## Claims

1. Use of a composition comprising :
(a) one or more derivatives of a pyrano [2,3 - d : 6,5 - d'] dipyrimidine , and
(b) one or more anti-retroviral drugs ,
for the manufacture of a medicament for the treatment of a retroviral infection in a mammal , (a) and (b) being in respective proportions such as to provide a synergistic effect against said retroviral infection .

2. Use of a composition comprising :
(a) one or more derivatives of pyrano [2,3 - d : 6,5 - d'] dipyrimidine , and
(b) one or more drugs effective against one or more retroviral or cellular proteins involved in the entry and/or replication of a retrovirus ,
for the manufacture of a medicine for the treatment of a retroviral infection in a mammal , in respective proportions such as to provide a synergistic effect in the said treatment .

3. Use of a composition comprising :
(a) one or more derivatives of a pyrano [2,3 - d : 6,5 - d'] dipyrimidine , and
(b) one or more anti-retroviral drugs , including drugs effective against one or more retroviral or cellular proteins involved in the entry and/or replication of a retrovirus ,
for the manufacture of a medicine for the treatment of a retroviral infection in a mammal .

4. A product containing:
(a) one or more derivatives of a pyrano [2,3 - d : 6,5 - d'] dipyrimidine , and
(b) one or more anti-retroviral drugs , including drugs effective against one or more retroviral or cellular proteins involved in the entry and/or replication of a retrovirus ,
in respective proportions such as to provide a synergistic effect against a retroviral infection in a mammal , as a combined preparation for simultaneous, separate or sequential use in retroviral infection therapy .

5. A product according to claim 4 , wherein said anti-retroviral drug (b) is a protease inhibitor .

6. A product according to claim 4 , wherein said anti-retroviral drug (b) is a reverse transcriptase inhibitor.

7. A product according to claim 4 , wherein said anti-retroviral drug (b) is an integrase inhibitor other than a derivative of a pyrano [2,3 - d : 6,5 - d'] di-pyrimidine .

8. A product according to any of claims 4 to 7 , in the form of a pharmaceutical preparation wherein at least one of (a) and (b) is in admixture with at least a pharmaceutically acceptable carrier.

9. Use of a derivative of a pyrano [2,3 - d : 6,5 - d'] dipyrimidine for the manufacture of a medicine for the treatment of a retroviral infection in a mammal .

10. Use according to claim 9 , wherein said treatment comprises inhibiting a retroviral enzyme .

11. Use according to claim 10 , wherein said retroviral enzyme is an integrase .

12. Use according to claim 10 , wherein the said retroviral enzyme is a reverse transcriptase .

13. A product according to any of claims 4 to 8 , wherein said retroviral infection is a lentiviral infection .

14. A product according to any of claims 4 to 8 , wherein said retroviral infection is a HIV infection .

15. A product according to any of claims 4 to 8 , wherein said derivative of pyrano [2,3 - d : 6,5 - d'] dipyrimidine is a 5-(4-substituted-phenyl)-5H-pyrano [2,3 - d : 6,5 - d'] dipyrimidine having the formula : 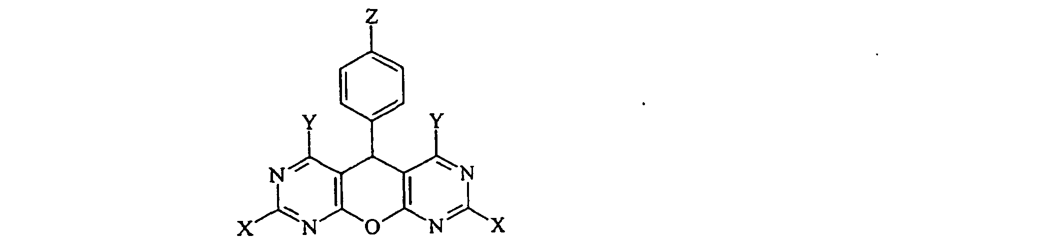 wherein :
- X is selected from hydroxy , halo and thiol ,
- Y is selected from hydroxy, methoxy and halo , and
- Z is selected from hydrogen , halo and nitro ,
a pharmaceutically acceptable addition salt or a stereochemically isomeric form thereof .

16. A product according to any of claims 4 to 8 , wherein the said derivative of pyrano [2,3-d : 6,5-d'] dipyrimidine is 5-(4-nitrophenyl)-2,8-dithiol-4,6-dihydroxy-5*H*-pyrano [2,3-d:6,5-d'] dipyrimidine .

17. Use according to any of claims 1 - 3 , wherein said anti-retroviral drug (b) is a protease inhibitor.

18. Use according to any of claims 1 - 3, wherein said anti-retroviral drug (b) is a reverse transcriptase inhibitor .

19. Use according to any of claims 1 - 3, wherein said anti-retroviral drug (b) is an integrase inhibitor other than a derivative of a pyrano [2,3-d:6,5-d'] dipyrimidine .

20. Use according to any of claims 1 - 3 and 9 - 12 , wherein said retroviral infection is a lentiviral infection .

21. Use according to any of claims 1 - 3 and 9 - 12 , wherein said retroviral infection is a HIV infection .

22. Use according to any of claims 1 - 3 and 9 -12 , wherein the said derivative of pyrano [2,3-d:6,5-d'] dipyrimidine is a 5-(4-substituted-phenyl)-5*H*-pyrano [2,3 - d : 6,5 - d'] dipyrimidine having the formula : 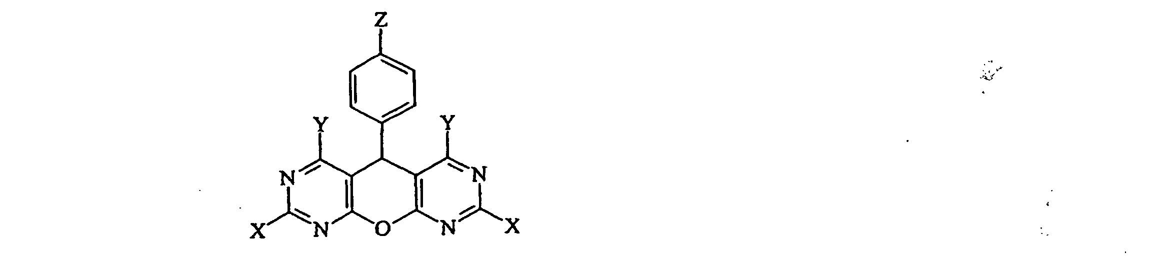 wherein :
- X is selected from hydroxy , halo and thiol ,
- Y is selected from hydroxy , methoxy and halo , and
- Z is selected from hydrogen , halo and nitro ,
a pharmaceutically acceptable addition salt or a stereochemically isomeric form thereof .

23. Use according to any of claims 1 - 3 and 9 - 12 , wherein the said derivative of pyrano [2,3-d:6,5-d'] dipyrimidine is 5-(4-nitrophenyl)-2,8-dithiol-4,6-dihydroxy-5*H*-pyrano [2,3-d:6,5-d'] dipyrimidine .

## Patentansprüche

1. Verwendung einer Zusammensetzung, die folgendes umfaßt:
(a) ein oder mehrere Derivate eines Pyrano[2,3-d:6,5-d']dipyrimidins und
(b) ein oder mehrere antiretrovirale Arzneistoffe
zur Herstellung eines Medikaments zur Behandlung einer retroviralen Infektion in einem Säugetier, wobei (a) und (b) jeweils in solchen Anteilen vorliegen, daß eine synergistische Wirkung gegen die retrovirale Infektion erzielt wird.

2. Verwendung einer Zusammensetzung, die folgendes umfaßt:
(a) ein oder mehrere Derivate eines Pyrano[2,3-d:6,5-d']dipyrimidins und
(b) ein oder mehrere Arzneistoffe, die gegen ein oder mehrere retrovirale oder zelluläre Proteine wirksam sind, die in das Eindringen und/oder die Replikation eines Retrovirus involviert sind,
zur Herstellung eines Medikamentes zur Behandlung einer retroviralen Infektion in einem Säugetier jeweils in solchen Anteilen, daß eine synergistische Wirkung bei der Behandlung erzielt wird.

3. Verwendung einer Zusammensetzung, die folgendes umfaßt:
(a) ein oder mehrere Derivate eines Pyrano[2,3-d:6,5-d']dipyrimidins und
(b) ein oder mehrere antiretrovirale Arzneistoffe, einschließlich von Arzneistoffen, die gegen ein oder mehrere retrovirale oder zelluläre Proteine wirksam sind, die in das Eindringen und/oder die Replikation eines Retrovirus involviert sind,
zur Herstellung eines Medikamentes zur Behandlung einer retroviralen Infektion in einem Säugetier.

4. Produkt, das folgendes enthält:
(a) ein oder mehrere Derivate eines Pyrano[2,3-d:6,5-d']dipyrimidins und
(b) ein oder mehrere antiretrovirale Arzneistoffe, einschließlich von Arzneistoffen, die gegen ein oder mehrere retrovirale oder zelluläre Proteine wirksam sind, die in das Eindringen und/oder die Replikation eines Retrovirus involviert sind, jeweils in solchen Verhältnissen, um eine synergistische Wirkung gegen eine retrovirale Infektion in einem Säugetier zu erzielen, als Kombinationspräparat zur simultanen, getrennten oder aufeinanderfolgenden Verwendung in der retroviralen Infektionstherapie.

5. Produkt nach Anspruch 4, wobei der antiretrovirale Arzneistoff (b) ein Proteaseinhibitor ist.

6. Produkt nach Anspruch 4, wobei der antiretrovirale Arzneistoff (b) ein Inhibitor der reversen Transkriptase ist.

7. Produkt nach Anspruch 4, wobei der antiretrovirale Arzneistoff (b) ein anderer IntegraseInhibitor als ein Derivat eines Pyrano[2,3-d:6,5-d']dipyrimidins ist.

8. Produkt nach einem der Ansprüche 4 bis 7 in Form einer pharmazeutischen Zubereitung, wobei zumindest eines von (a) und (b) in Mischung mit zumindest einem pharmazeutisch verträglichen Träger vorliegt.

9. Verwendung eines Derivats eines Pyrano[2,3-d:6,5-d']dipyrimidins zur Herstellung eines Medikamentes zur Behandlung einer retroviralen Infektion in einem Säugetier.

10. Verwendung nach Anspruch 9, wobei die Behandlung die Hemmung eines retroviralen Enzyms umfaßt.

11. Verwendung nach Anspruch 10, wobei das retrovirale Enzym eine Integrase ist.

12. Verwendung nach Anspruch 10, wobei das retrovirale Enzym eine reverse Transkriptase ist.

13. Produkt nach einem der Ansprüche 4 bis 8, wobei die retrovirale Infektion eine lentivirale Infektion ist.

14. Produkt nach einem der Ansprüche 4 bis 8, wobei die retrovirale Infektion eine HIV-Infektion ist.

15. Produkt nach einem der Ansprüche 4 bis 8, wobei das Derivat von Pyrano[2,3-d:6,5-d']dipyrimidin ein 5-(4-substituiertes Phenyl)-5H-pyrano[2,3-d:6,5-d']dipyrimidin mit der folgenden Formel: 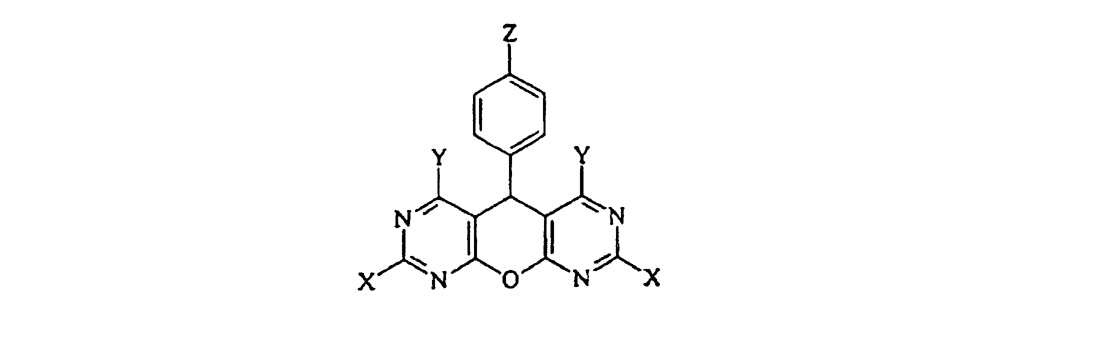 wobei:
- X aus Hydroxy, Halo und Thiol ausgewählt ist,
- Y aus Hydroxy, Methoxy und Halo ausgewählt ist und
- Z aus Wasserstoff, Halo und Nitro ausgewählt ist,
ein pharmazeutisch verträgliches Additionssalz oder eine stereochemisch isomere Form hiervon ist.

16. Produkt nach einem der Ansprüche 4 bis 8, wobei das Derivat von Pyrano[2,3-d:6,5-d'] dipyrimidin 5-(4-Nitrophenyl)-2,8-dithiol-4,6-dihydroxy-5H-pyrano[2,3-d:6,5-d'] dipyrimidin ist.

17. Verwendung nach einem der Ansprüche 1-3, wobei der antiretrovirale Arzneistoff (b) ein Proteaseinhibitor ist.

18. Verwendung nach einem der Ansprüche 1-3, wobei der antiretrovirale Arzneistoff (b) ein reverser Transkriptase-Inhibitor ist.

19. Verwendung nach einem der Ansprüche 1-3, wobei der antiretrovirale Arzneistoff (b) ein anderer Integraseinhibitor als ein Derivat eines Pyrano[2,3-d:6,5-d']dipyrimidins ist.

20. Verwendung nach einem der Ansprüche 1-3 und 9-12, wobei die retrovirale Infektion eine lentivirale Infektion ist.

21. Verwendung nach einem der Ansprühe 1-3 und 9-12, wobei die retrovirale Infektion eine HIV-Infektion ist.

22. Verwendung nach einem der Ansprüche 1-3 und 9-12, wobei das Derivat eines Pyrano[2,3-d:6,5-d']dipyrimidins ein 5-(4-substituiertes Phenyl)-5H-pyrano[2,3-d:6,5-d']dipyrimidin mit der folgenden Formel: 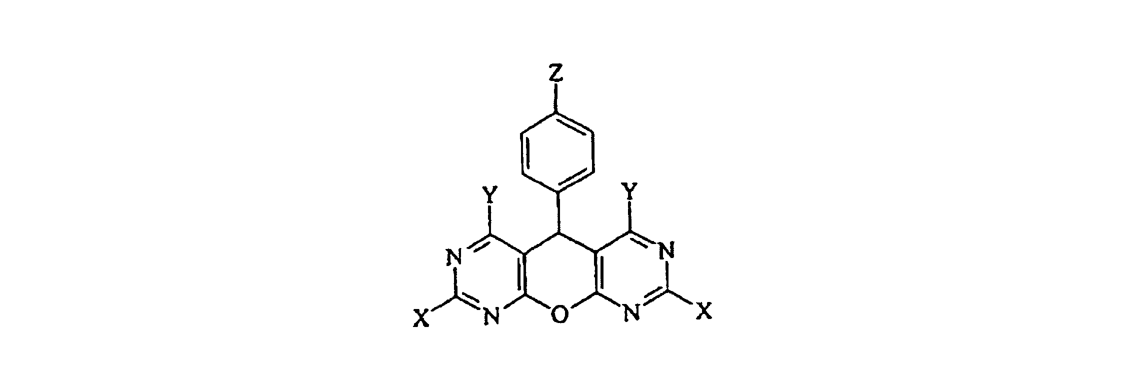 wobei:
- X aus Hydroxy, Halo und Thiol ausgewählt ist,
- Y aus Hydroxy, Methoxy und Halo ausgewählt ist und
- Z aus Wasserstoff, Halo und Nitro ausgewählt ist,
ein pharmazeutisch verträgliches Additionssalz oder eine stereochemisch isomere Form hiervon ist.

23. Verwendung nach einem der Ansprüche 1-3 und 9-12, wobei das Derivat eines Pyrano[2,3-d:6,5-d']dipyrimidins 5-(4-substituiertes Nitrophenyl)-2,8-dithiol-4,6-dihydroxy-5H-pyrano[2,3-d:6,5-d']dipyrimidin ist.

## Revendications

1. Utilisation d'une composition comprenant :
(a) un ou plusieurs dérivés d' une pyrano [2 , 3 - d : 6 , 5 - d'] dipyrimidine et
(b) un ou plusieurs médicaments anti-rétroviraux ,
pour la fabrication d' un médicament pour le traitement d' une infection rétrovirale chez un mammifère , (a) et (b) étant en proportions respectives propres à procurer un effet synergistique contre ladite infection rétrovirale .

2. Utilisation d'une composition comprenant :
(a) un ou plusieurs dérivés de pyrano [2 , 3 - d : 6 , 5 - d'] dipyrimidine , et
(b) un ou plusieurs médicaments effectifs contre une ou plusieurs protéines rétrovirales ou cellulaires impliquées dans l' entrée et / ou la réplication d' un rétrovirus ,
pour la fabrication d' un médicament pour le traitement d' une infection rétrovirale chez un mammifère , en proportions respectives propres à procurer un effet synergistique dans ledit traitement .

3. Utilisation d'une composition comprenant :
(a) un ou plusieurs dérivés d' une pyrano [2 , 3 - d : 6 , 5 - d'] dipyrimidine et
(b) un ou plusieurs médicaments anti-rétroviraux , comprenant des médicaments effectifs contre une ou plusieurs protéines rétrovirales ou cellulaires impliquées dans l' entrée et / ou la réplication d' un rétrovirus ,
pour la fabrication d' un médicament pour le traitement d' une infection rétrovirale chez un mammifère .

4. Un produit contenant :
(a) un ou plusieurs dérivés d' une pyrano [2 , 3 - d : 6 , 5 - d'] dipyrimidine , et
(b) un ou plusieurs médicaments anti-rétroviraux , comprenant des médicaments effectifs contre une ou plusieurs protéines rétrovirales ou cellulaires impliquées dans l' entrée et / ou la réplication d' un rétrovirus ,
en proportions respectives propres à procurer un effet synergistique contre une infection rétrovirale chez un mammifère , en tant que préparation combinée pour utilisation simultanée , séparée ou séquentielle dans une thérapie d' infection rétrovirale .

5. Un produit selon la revendication 4 , dans lequel ledit médicament anti-rétroviral (b) est un inhibiteur de protéase .

6. Un produit selon la revendication 4 , dans lequel ledit médicament anti-rétroviral (b) est un inhibiteur de transcriptase inversée .

7. Un produit selon la revendication 4 , dans lequel ledit médicament anti-rétroviral (b) est un inhibiteur d' intégrase autre qu' un dérivé de pyrano [2,3 - d : 6 , 5 - d'] di-pyrimidine .

8. Un produit selon l' une quelconque des revendications 4 à 7 , sous la forme d' une préparation pharmaceutique dans laquelle l' un au moins de (a) et (b) est en mélange avec au moins un support pharmaceutiquement acceptable .

9. Utilisation d'une pyrano [2 , 3 - d : 6 , 5 - d'] dipyrimidine pour la fabrication d' un médicament pour le traitement d' une infection rétrovirale chez un mammifère .

10. Utilisation selon la revendication 9 , dans laquelle ledit traitement comprend l'inhibition d' une enzyme rétrovirale .

11. Utilisation selon la revendication 10 , dans laquelle ladite enzyme rétrovirale est une intégrase .

12. Utilisation selon la revendication 10 , dans laquelle ladite enzyme rétrovirale est une transcriptase inversée .

13. Un produit selon l'une quelconque des revendications 4 à 8 , dans lequel ladite infection rétrovirale est une infection lentivirale .

14. Un produit selon l' une quelconque des revendications 4 à 8 , dans lequel ladite infection rétrovirale est une infection par le VIH .

15. Un produit selon l' une quelconque des revendications 4 à 8 , dans lequel ledit dérivé de pyrano [2 , 3 - d : 6 , 5 - d'] dipyrimidine est une 5-(4-phényl substitué)-5*H*-pyrano [2 , 3 - d : 6 , 5 - d'] dipyrimidine ayant la formule : 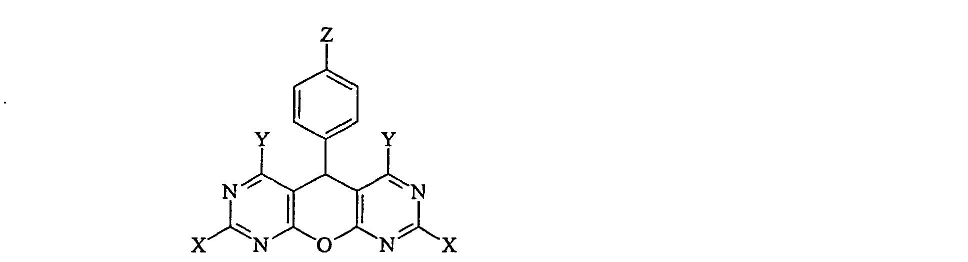 dans laquelle :
- X est sélectionné parmi hydroxy , halo et thiol ,
- Y est sélectionné parmi hydroxy , méthoxy et halo , et
- Z est sélectionné parmi hydrogène , halo et nitro ,
un sel d' addition pharmaceutiquement acceptable ou une forme stéréo-chimiquement isomère de celle-ci .

16. Un produit selon l'une quelconque des revendications 4 à 8 , dans lequel ledit dérivé de pyrano [2 , 3 - d : 6 , 5 - d'] dipyrimidine est la 5-(4-nitro-phényl)-2,8-dithiol-4,6-dihydroxy-5H-pyrano [2 , 3 - d : 6 , 5 - d'] dipyrimidine .

17. Utilisation selon l' une quelconque des revendications 1 - 3 , dans laquelle ledit médicament anti-rétroviral (b) est un inhibiteur de protéase .

18. Utilisation selon l' une quelconque des revendications 1 - 3 , dans laquelle ledit médicament anti-rétroviral (b) est un inhibiteur de transcriptase inversée .

19. Utilisation selon l' une quelconque des revendications 1 - 3 , dans laquelle ledit médicament anti-rétroviral (b) est un inhibiteur d' intégrase autre qu' un dérivé de pyrano [2 , 3 - d : 6 , 5 - d'] dipyrimidine .

20. Utilisation selon l'une quelconque des revendications 1 - 3 et 9 - 12 , dans laquelle ladite infection rétrovirale est une infection lentivirale .

21. Utilisation selon l' une quelconque des revendications 1 - 3 et 9 - 12 , dans laquelle ladite infection rétrovirale est une infection par le VIH .

22. Utilisation selon l'une quelconque des revendications 1 - 3 et 9 - 12 , dans laquelle ledit dérivé de pyrano [2 , 3 - d : 6 , 5 - d'] dipyrimidine est une 5-(4-phényl substitué)-5*H*-pyrano [2 , 3 - d : 6 , 5 - d'] dipyrimidine ayant la formule: 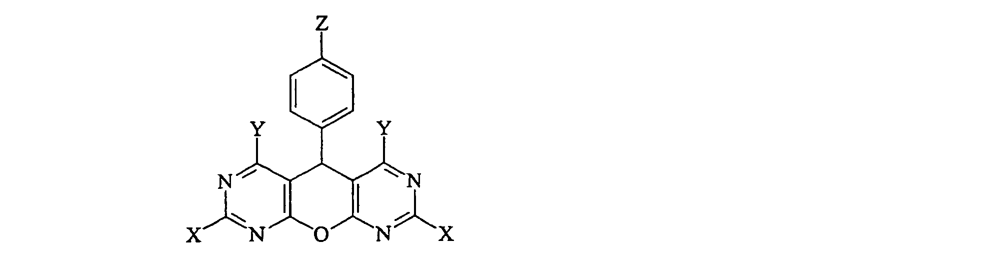 dans laquelle :
- X est sélectionné parmi hydroxy , halo et thiol ,
- Y est sélectionné parmi hydroxy , méthoxy et halo , et
- Z est sélectionné parmi hydrogène , halo et nitro ,
un sel d' addition pharmaceutiquement acceptable ou une forme stéréo-chimiquement isomère de celle-ci .

23. Utilisation selon l' une quelconque des revendications 1 - 3 et 9 - 12 , dans laquelle ledit dérivé de pyrano [2 , 3 - d : 6 , 5 - d'] dipyrimidine est la 5-(4-nitrophényl)-2,8-dithiol-4,6-dihydroxy-5H-pyrano [2, 3 - d : 6 , 5 - d'] dipyrimidine .
